# EUROPÄISCHE PATENTSCHRIFT

(11) **EP 1 330 474 B1**
(45) Veröffentlichungstag und Bekanntmachung des Hinweises auf die Patenterteilung: **20.02.2008**
(21) Anmeldenummer: 01990381.4
(22) Anmeldetag: 01.11.2001
(51) Int. Cl.: G01N 33/68

(54) **DIAGNOSTISCHE VERWENDUNG EINES SPEZIFISCHEN CO-AKTIVATORS FÜR HUMANE NUKLEÄRE REZEPTOREN**
DIAGNOSTIC USE OF A SPECIFIC CO-ACTIVATOR FOR HUMAN NUCLEAR RECEPTORS
UTILISATION A DES FINS DE DIAGNOSTIC D'UN CO-ACTIVATEUR SPECIFIQUE DESTINE AUX RECEPTEURS NUCLEAIRES HUMAINS

(30) Priorität: 03.11.2000 DE 10054639
(43) Veröffentlichungstag der Anmeldung: 30.07.2003
(73) Patentinhaber: Bayer Schering Pharma Aktiengesellschaft, 13353 Berlin (DE)
(72) Erfinder: OBENDORF, Maik, 99423 Weimar (DE); WOLF, Siegmund, 07745 Jena (DE)
(74) Vertreter: Geyer, Fehners & Partner
(86) Internationale Anmeldenummer: PCT/EP2001/012668
(87) Internationale Veröffentlichungsnummer: WO 2002/036621

(56) Entgegenhaltungen:
- WO-A-00/12544
- WO-A-98/12327
- NAKAMURA M ET AL: "WHEN OVEREXPRESSED, A NOVEL CENTROSOMAL PROTEIN, RANBPM, CAUSES ECTOPIC MICROTUBULE NUCLEATION SIMILAR TO GAMMA-TUBULIN" THE JOURNAL OF CELL BIOLOGY, ROCKEFELLER UNIVERSITY PRESS, US, Bd. 143, Nr. 4, 16. November 1998 (1998-11-16), Seiten 1041-1052, XP000872664 ISSN: 0021-9525 in der Anmeldung erwähnt
- MCKENNA N J ET AL: "NUCLEAR RECEPTOR COREGULATORS: CELLULAR AND MOLECULAR BIOLOGY" ENDOCRINE REVIEWS, BALTIMORE, MD, US, Bd. 20, Nr. 3, Juni 1999 (1999-06), Seiten 321-344, XP002921971
- DING XIU FEN ET AL: "Nuclear receptor-binding sites of coactivators glucocorticoid receptor interacting protein 1 (GRIP1) and steroid receptor coactivator 1 (SRC-1): Multiple motifs with different binding specificities." MOLECULAR ENDOCRINOLOGY, Bd. 12, Nr. 2, Februar 1998 (1998-02), Seiten 302-313, XP001040351 ISSN: 0888-8809
- NISHITANI H ET AL: "Full-sized RanBPM cDNA encodes a protein possessing a long stretch of proline and glutamine within the N-terminal region, comprising a large protein complex" GENE: AN INTERNATIONAL JOURNAL ON GENES AND GENOMES, ELSEVIER SCIENCE PUBLISHERS, BARKING, GB, Bd. 272, Nr. 1-2, 11. Juli 2001 (2001-07-11), Seiten 25-33, XP004274836 ISSN: 0378-1119

## Beschreibung

In der Erfindung wird ein geklontes Gen beschrieben, welches für ein Protein kodiert, das beim Menschen als Co-Aktivator von Hormonrezeptoren - insbesondere des Androgenrezeptors - fungiert, sowie die Anwendung dieses Co-Aktivatorproteins als wichtiger Bestandteil von klinischen Tests zur Diagnostizierung von Erkrankungen beim Menschen.

Die Superfamilie der nukleären Rezeptoren (NR), zu der mehr als 50 verschiedene Proteine gehören, ist eine Gruppe verwandter Transkriptionsfaktoren, die die Transkription des jeweiligen Zielgens als Reaktion auf spezifische Liganden steuern. Die Familie kann nach bestimmten Charakteristika wie z.B. Dimerisationsstatus, Art des Liganden oder Struktur des DNA-Reaktionselements, in mehrere Subfamilien unterteilt werden (Beato et al., 2000, Human Reproduct. Update, 6, 225-236). Charakteristisches Merkmal der NR ist die übereinstimmende Struktur der funktionellen Domänen (mit den Bezeichnungen A bis F) mit einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Reaktionselementen verantwortlich ist und aus zwei Zinkfinger-Motiven besteht, einer variablen Scharnierdomäne und einer konservierten multifunktionalen C-terminalen Ligandenbindungsdomäne (LBD) mit Dimerisations- und Ligandenabhängiger Transaktivierungsfunktion (AF-2). Im Anschluss daran folgt die am weitesten C-terminal gelegene Region, deren Funktion nicht bekannt ist und die bei Rezeptoren wie z.B. PPAR (Peroxisomproliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige NR (z.B. AR) wurde nachgewiesen, dass die N-terminale Region in der Lage ist, mit der C-terminalen Region zu interagieren (Brinkmann et al., 1999, J. Steroid Biochem. and Mol. Biol., 69, 307-313). Steroidhormonrezeptoren wie z.B. Estrogen- (ER), Progesteron- (PR), Glukokortikoid- (GR), Mineralokortikoid- (MR) und Androgenrezeptoren (AR) binden steroidale Liganden, die sich von Pregnenolon ableiten wie die Gestagene, die Estrogene, die Glukokortikoide, Mineralokortikoide und die Androgene. Die Ligandenbindung aktiviert den Rezeptor und steuert die Expression entsprechender Zielgene.

Darüber hinaus wurde eine weitere Proteinklasse, die sog. Co-Modulatoren, als Klasse von Substanzen identifiziert, die bei der Aktivierung (Co-Aktivatoren) bzw. Repression (Co-Repressoren) der Gentranskription als Brückenmoleküle zwischen dem Transkriptionsinitiationskomplex und den NR dienen (McKenna et al., 1999, Endocr. Rev., 20, 321-347). Ein Co-Aktivator muss fähig sein, die Rezeptorfunktion zu verstärken und agonistenabhängig - aber nicht in Anwesenheit eines Antagonisten - mit der Aktivierungsdomäne von NR direkt zu interagieren. Er muss auch mit dem basalen Transkriptionsapparat interagieren, und schließlich darf er nicht selbst die basale Transkriptionsaktivität verstärken. Die meisten Co-Modulatoren interagieren mit Hilfe eines oder mehrerer LXXLL-Motive (NR-Boxes) mit der AF-2-Domäne von NR, jedoch wurden auch einige Co-Modulatoren beschrieben, die mit anderen NR-Regionen interagieren (Ding et al., 1998, Mol. Endocrinol., 12, 302-313). Darüber hinaus interagieren viele identifizierte Co-Modulatoren in ähnlicher Weise mit mehreren verschiedenen NR, so dass der Spezifitätsgrad jedes isolierten Co-Modulators getestet werden muss. Um neue potenzielle Co-Aktivatoren zu identifizieren, wurde eine cDNA-Bibliothek aus fetalem Gehirn mit Fragmenten des Androgenrezeptors (AR) als Sonden gescreent. Dies geschah mit Hilfe des Hefe-zwei Hybrid-Systems.

Die WO 00/01813 beschreibt nicht-androgene Ligandenbindungspeptide, die sich von den Aminosäuren 234-391 des Androgenrezeptors ableiten sowie deren Verwendung zur Herstellung eines Medikaments zur Behandlung androgenbedingter Erkrankungen.

Mit der Erfindung wird ein spezifischer Co-Modulator für den humanen AR isoliert mit einer Interaktion zwischen AR und RanBPM.

Das Gen für den Co-Modulator (RanBPM) wird als ein aus 500 Aminosäureresten bestehendes Protein mit einem Molekulargewicht von 55 kD beschrieben, das in dem an der Nukleation der Mikrotubuli beteiligten Zentrosom lokalisiert ist (GenBank: NM_005493) (Nakamura et al., 1998, J. Cell. Biol., 143, 1041-1052).

Die vorliegende Erfindung ermöglicht durch die Klonierung und Reproduktion des AR-Aktivatorgens die Entwicklung neuer Labortests zur Prüfung der Androgenspezifizität potenzieller therapeutisch wirksamer Substanzen.

Die vorliegende Erfindung stellt ein Verfahren zur Testung des hormonellen, insbesondere des androgenen und antiandrogenen in-vitro-Effekts einer chemischen Substanz bereit, das die Schritte umfaßt
a) Transformationen von Wirtszellen mit Hilfe eines genetischen Konstrukts, welches bewirkt, daß die Wirtszellen künftig sowohl humanes nukleäres Rezeptorprotein, ausgewählt aus der Gruppe bestehend aus Androgenrezeptor (AR), Estrogenrezeptor α (ERα), Estrogenrezeptor β (ERβ), Progesteronrezeptor A (PRA), Progesteronrezeptor B (PRB), Glukokortikoidrezeptor (GR) und Mineralkortikoidrezeptor (MR), als auch RanBPM (Genbank NM_005493)-Protein produzieren,
b) Exposition der transformierten Wirtszellen gegenüber der chemischen Substanz und
c) Messung der Transkriptionsaktivität des nukleären Rezeptors in Anwesenheit von RanBPM.

Weitere Ziele, Vorteile und Eigenschaften der vorliegenden Erfindung sind aus der folgenden Spezifikation ersichtlich.

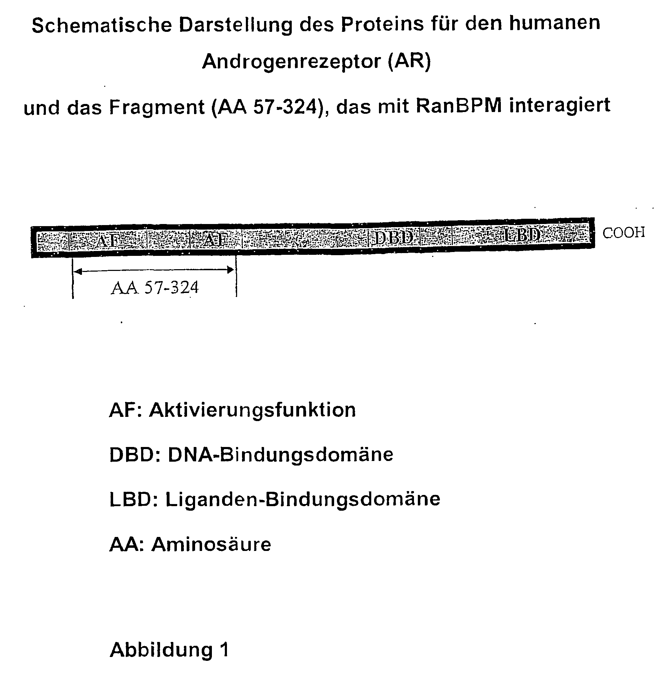

Abbildung 1/1 enthält eine schematische Darstellung des AR mit Kennzeichnung der von AS 57 bis AS 324 reichenden AR-Domäne, die zur Interaktion mit RanBPM fähig ist.

Die vorliegende Erfindung wird durch die Isolierung eines neuen Steuerungsproteins beim Menschen ermöglicht. Bei diesem Steuerungsprotein handelt es sich um das NR-assoziierte Protein, das hier als RanBPM bezeichnet wird und einen Co-Mediator, insbesondere einen Co-Aktivator für den AR und weitere nukleäre Rezeptoren darstellt, z.B. für die Rezeptoren ER, PR, GR, MR, TR (Schilddrüsenhormon-Rezeptor), VDR (Vitamin-D-Rezeptor), PPAR, RAR (Retinoid-A-Rezeptor) und RXR. RanBPM ist ein Protein, das als spezifischer Co-Mediator fungieren kann, indem es den Transkriptionseffekt der Bindung von Hormonen an den nukleären Rezeptor verstärkt oder reprimiert und darüber hinaus die Bindung und Aktivierung des nukleären Rezeptors durch Moleküle fördert, denen früher keine hormonelle Wirkung zugeschrieben wurde.

Im Folgenden wird die vorliegende Erfindung anhand von AR als Beispiel veranschaulicht.

Mit Hilfe des zweifach hybridisierten Hefe-Systems wurde eine cDNA-Bibliothek aus fetalem Gehirn mit dem humanen AR-Fragment, das für die Aminosäuren 57 bis 324 kodiert, als Sonde gescreent (Abbildung 1). Ein beim Screenen isolierter cDNA-Klon war nicht von einem bereits früher identifizierten cDNA-Klon (RanBPM) unterscheidbar (Genbank-Zugangsnummer NM_005493; Nakamura et al. 1998, J. Cell. Biol., 143, 1041-1052).

Es wird insbesondere erwartet, dass RanBPM ein besonderes Anwendungsgebiet als Bestandteil eines Arzneimittel-Prüf- oder - Screeningprotokolls haben wird. Bei der Evaluation neuer klinischer Substanzen für die pharmazeutische Anwendung ist es allgemein üblich, die Substanzen auf eine eventuelle hormonelle Aktivität zu prüfen. Bei der Verabreichung pharmakologisch wirksamer Substanzen sind androgene oder antiandrogene Nebenwirkungen in manchen Fällen von Bedeutung. Zur Prüfung der androgenen Aktivität dienten früher Verfahren, bei denen die Fähigkeit zur Bindung und Aktivierung der Transkriptionsaktivität der Androgenrezeptoren gemessen wurde.

Das RanBPM-Molekül könnte aber nicht nur zur Testung von Substanzen auf ihre potenzielle Eignung zur pharmazeutischen Anwendung, sondern auch zur Testung nicht-pharmazeutischer Substanzen auf eine potenzielle androgene bzw. antiandrogene Wirkung nützlich sein. Denn von vielen, in geringen Mengen in der Umwelt vorhandenen Verunreinigungen wird angenommen, dass sie bei verschiedenen Teilen der Bevölkerung eine androgene/antiandrogene bzw. estrogene/antiestrogene Aktivität aufweisen.

Um Substanzproben auf ihre eventuelle androgene/antiandrogene Wirksamkeit zu prüfen, könnten genetische Konstrukte wie Expressionskassetten sowohl für den Androgenrezeptor als auch für RanBPM in vitro in eukaryote Wirtszellen transformiert werden, z.B. in Prostata-, Nerven- oder Muskelzelllinien. Dabei könnte gleichzeitig auch ein leicht nachweis- und quantifizierbares Detektorgen in die Zellen transformiert werden. Ein geeignetes Detektorgen (Reportergen) wäre das für Luciferase, für CAT (Chloramphenicolazetyltransferase), für β-Galaktosidase oder für ähnliche Systeme kodierende Gen, dessen Expression sich photometrisch nachweisen lässt. Die Zellen werden dann der Einwirkung des Arzneimittels bzw. des aus der Umwelt stammenden Testmaterials ausgesetzt. Material mit androgener/antiandrogener Aktivität ist dann an der erhöhten bzw. verminderten Aktivität des Reportergens erkennbar. Möglich wäre auch die Messung des Einflusses von Testmaterial auf die AR/RanBPM-Interaktion durch doppelt-hybride Systeme, Immunpräzipitation, GST-Pull-down-Assays, Gelretardationsassays, FRET-Analayse, ABCD-Assays etc.

### BEISPIEL

Unter Verwendung einer cDNA-Library aus fetalem Gehirn und eines humanen AR-Fragments, das für die Aminosäuren 57 bis 324 kodiert, als Sonde (Abbildung 1) wurde ein Screening mittels des Hefe-zwei Hybrid-Systems durchgeführt. In Übereinstimmung mit den Anweisungen des Herstellers (Clontech) betrug die Zahl der gescreenten Klone 3x10⁶, entsprach also fast der Zahl unabhängiger Klone laut Angaben des Herstellers (3,5x10⁶). Davon wurden 500 positive Klone ausgewählt und mit einem β-Galaktosidase-Assay getestet; 200 wurden als IacZ-positive Klone bestätigt. Die Inserts dieser Klone wurden durch PCR amplifiziert. Mittels Restriktionsfragmentanalysen und Sequenzierung wurden mindestens 17 verschiedene Klone identifiziert. Einer davon war ein Klon mit einem 2500bp-Insert, das für den gesamten ORF (Open reading frame) von RanBPM kodiert (Genbank-Zugangsnummer NM_005493). Dieser Klon wurde beim Screening der Bibliothek einmal identifiziert.

Hier weisen wir mittels zweifach hybridisiertem Hefe-System nach, dass RanBPM an humane AR bindet. Damit wird erstmals die Co-Aktivatorfunktion des RanBPM-Proteins für Steroidrezeptoren (z.B. AR) nachgewiesen. Diese Interaktion eröffnet die Möglichkeit einer spezifischen therapeutischen Intervention bei verschiedenen Indikationen, an deren Pathogenese oder Therapie Hormone beteiligt sind.

## Patentansprüche

1. Verfahren zur Testung des hormonellen, insbesondere des androgenen und antiandrogenen in-vitro-Effekts einer chemischen Substanz, das die Schritte umfaßt
a) Transformationen von Wirtszellen mit Hilfe eines genetischen Konstrukts, welches bewirkt, daß die Wirtszellen künftig sowohl humanes nukleäres Rezeptorprotein, ausgewählt aus der Gruppe bestehend aus Androgenrezeptor (AR), Estrogenrezeptor α (ERα), Estrogenrezeptor β (ERβ), Progesteronrezeptor A (PRA), Progesteronrezeptor B (PRB), Glukokortikoidrezeptor (GR) und Mineralkortikoidrezeptor (MR), als auch RanBPM (Genbank NM_005493)-Protein produzieren,
b) Exposition der transformierten Wirtszellen gegenüber der chemischen Substanz und
c) Messung der Transkriptionsaktivität des nukleären Rezeptors in Anwesenheit von RanBPM.

2. Verfahren nach Anspruch 1, **dadurch gekennzeichnet, daß** eukaryote Zellen als Wirtszellen verwendet werden, wobei die Zellen Keine menschlichen embryonalen Zellen sind.

3. Verfahren nach Anspruch 2, **dadurch gekennzeichnet, daß** Prostatazellen, Nervenzellen oder Muskelzellen als eukaryote Zellen verwendet werden.

4. Verfahren nach einem der Ansprüche 1 bis 3, **dadurch gekennzeichnet, daß** ein Arzneimittel als chemische Substanz verwendet wird.

## Claims

1. Method for testing the hormonal, in particular the androgenic and antiandrogenic, in vitro effect of a chemical substance comprising the steps of
a) transforming host cells with a genetic construct effective in the host cells to produce, in future, both human nuclear receptor protein selected from the group consisting of androgen receptor (AR), estrogen receptor α (ERα), estrogen receptor β (ERβ), progesterone receptor A (PRA), progesterone receptor B (PRB), glucocorticoid receptor (GR) and mineralocorticoid receptor (MR), and RanBPM (Genbank NM_005493) protein,
b) exposing the transformed host cells to the chemical substance and
c) measuring the level of transcriptional activity of the nuclear receptor in the presence of RanBPM.

2. Method according to Claim 1, **characterized in that** the host cells used are eucaryotic cells, wherein the cells are not human embryonal cells.

3. Method according to Claim 2, **characterized in that** the eucaryotic cells used are prostate cells, neuronal cells or muscle cells.

4. Method according to any of Claims 1 to 3, chatracterized in that the chemical substance used is a pharmaceutical agent.

## Revendications

1. Procédé de test de l'effet hormonal in vitro, en particulier de l'effet androgène in vitro et de l'effet antiandrogène in vitro, d'une substance chimique, comportant les étapes consistant à :
a) transformer des cellules hôtes à l'aide d'une construction génétique, laquelle a pour conséquence que les cellules hôtes produisent dès lors aussi bien une protéine de récepteur nucléaire humaine, sélectionnée parmi le groupe constitué du récepteur des androgènes (AR), du récepteur α des estrogènes (ERα), du récepteur β des oestrogènes (ERβ), du récepteur A de la progestérone (PRA), du récepteur B de la progestérone (PRB), du récepteur des glucocorticoïdes (GR) et du récepteur des minéralocorticoïdes (MR), qu'une protéine RanBPM (Genbank NM_005493),
b) exposer les cellules hôtes transformées à la substance chimique et
c) mesurer l'activité transcriptionnelle du récepteur nucléaire en présence de RanBPM.

2. Procédé selon la revendication 1, **caractérisé en ce que** des cellules eucaryotes sont utilisées en tant que cellules hôtes, les cellules n'étant pas des cellules embryonnaires humaines.

3. Procédé selon la revendication 2, **caractérisé en ce que** des cellules de prostate, des cellules nerveuses ou des cellules musculaires sont utilisées en tant que cellules eucaryotes.

4. Procédé selon l'une quelconque des revendications 1 à 3, **caractérisé en ce qu'**un médicament est utilisé en tant que substance chimique.
